# EUROPÄISCHE PATENTSCHRIFT

(11) **EP 0 544 158 B1**
(45) Veröffentlichungstag und Bekanntmachung des Hinweises auf die Patenterteilung: **02.10.1996**
(21) Anmeldenummer: 92119414.8
(22) Anmeldetag: 13.11.1992
(51) Int. Cl.: C08F 26/06, A61K 7/06

(54) **Verwendung von Homo- oder Copolymerisaten auf Basis von quaternisierten 1-Vinylimidazolen als organische Polyelektrolyte**
Use of homo- or co-polymers based on quaternised 1-vinyl imidazoles as organic polyelectrolyte
Usage de homo- ou copolymères à base de 1-vinylimidazoles quaternisé comme polyélectrolytes organiques

(30) Priorität: 26.11.1991 DE 4138763
(43) Veröffentlichungstag der Anmeldung: 02.06.1993
(73) Patentinhaber: BASF Aktiengesellschaft, 67063 Ludwigshafen (DE)
(72) Erfinder: Meyer, Harald, Dr., W-6705 Deidesheim (DE); Sanner, Axel, Dr., W-6710 Frankenthal (DE); Reinhardt, Rolf-Dieter, Dr., W-6711 Laumersheim (DE); Frosch, Franz, Dr., W-6702 Bad Duerkheim (DE); Raubenheimer, Hans-Jürgen, 6834 Ketsch (DE)

(56) Entgegenhaltungen:
- EP-A- 0 162 388
- DE-A- 3 617 069
- FR-A- 1 089 847
- GB-A- 2 161 172

## Beschreibung

Die vorliegende Erfindung betrifft die Verwendung von Homo- oder Copolymerisaten auf Basis von quaternisierten 1-Vinyl-imidazolen als organische Polyelektrolyte in kosmetischen Zubereitungen, in Mitteln zur leitfähigkeitserhöhung, in Flockungsmitteln und in Hilfsmitteln bei der Erdölgewinnung.

Aus der DE-C 36 17 069 (1) ist bekannt, daß 1-Vinylimidazole mit Methylchlorid in Wasser quaternisierbar sind. Hieraus erhält man Homo- und Copolymerisate, die eine hohe Ladungsdichte und Hydrolysestabilität aufweisen, aber korrosiv sind und deshalb nur in entsprechend ausgerüsteten Apparaturen und Behältnissen gehandhabt werden können.

Aus der US-A 3 910 862 (2) sind kationische Copolymerisate auf Basis von Vinylpyrrolidon und mit Dimethylaminoethylmethacrylat bekannt, die nicht korrosiv, dafür aber nur eine geringe Ladungsdichte aufweisen und nicht hydrolysestabil sind.

In der WO 90/01920 (3) wird ein Copolymerisat beschrieben, das aus Vinylpyrrolidon und mit Dimethylsulfat quaternisiertem Dimethylaminopropylmethacrylamid aufgebaut ist. Dieses Copolymerisat ist ebenfalls nicht korrosiv, dafür aber hydrolysestabil. Die Ladungsdichte ist jedoch gering.

Aufgabe der vorliegenden Erfindung war es, ein Polymerisat bereit zustellen, welches als organischer Polyelektrolyt für die genannten Anwendungsgebiete geeignet ist und die drei Eigenschaften der fehlenden Korrosivität, der hohen Ladungsdichte und der Hydrolysestabilität in sich vereint.

Demgemäß wurde die Verwendung von Homo- oder Copolymerisaten auf Basis von quaternisierten 1-Vinylimidazolen, welche aus
A) 10 bis 100 Gew.-% eines 1-Vinylimidazols der allgemeinen Formel I 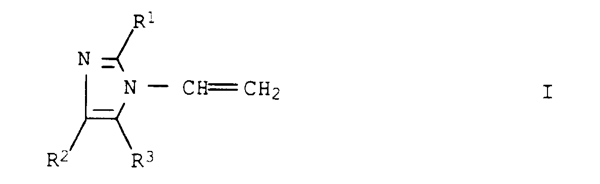 in der R¹ bis R³ für Wasserstoff oder C₁- bis C₄-Alkyl stehen, und
B) 0 bis 90 Gew.-% weiterer copolymerisierbarer Monomerer, jeweils bezogen auf die Summe aus A in guaternisierter Form und B,
aufgebaut sind, als organische Polyelektrolyte in kosmetischen Zubereitungen, in Mitteln zur Leitfähigkeitserhöhung, in Flockungsmitteln und in Hilfsmitteln bei der Erdölgewinnung gefunden, welche dadurch gekennzeichnet ist, daß man solche Homo- oder Copolymerisate einsetzt, bei deren Herstellung als Quaternierungsmittel Verbindungen der allgemeinen Formel II

(R⁴) ₙA II

in der
- R⁴: für C₁- bis C₂₀-Alkyl oder Benzyl steht,
- A: Jodid, Carbonat, Methylsulfat, Ethylsulfat oder Sulfat bezeichnet und
- n: die Zahl 1 oder 2 bedeutet,
verwendet wurden.

Als Reste R¹ bis R³ am 1-Vinylimidazol-System I kommen vor allem Methyl und Ethyl, daneben aber auch n-Propyl, iso-Propyl, n-Butyl, iso-Butyl, sec.-Butyl und tert.-Butyl in Betracht. Besonders bevorzugt wird jedoch unsubstituiertes 1-Vinylimidazol, d.h. R¹ bis R³ stehen für Wasserstoff.

Als Reste R⁴ in den Quaternierungsmitteln II kommen neben den bereits oben als Vertreter für C₁- bis C₄-Alkyl genannten Resten weiterhin vor allem n-Amyl, n-Hexyl, n-Heptyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Hexadecyl, n-Octadecyl und n-Eicosyl sowie Benzyl in Betracht. Besonders bevorzugt werden hiervon jedoch Methyl und Ethyl.

Als Quaternierungsmittel II selbst eignen sich vor allem Methyljodid, Dimethylsulfat und Diethylsulfat, daneben können aber beispielsweise auch Dimethylcarbonat, Diethylcarbonat, Benzyljodid oder längerkettige Alkyljodide wie Decyljodid, Dodecyljodid, Tetradecyljodid, Hexadecyljodid oder Octadecyljodid mit gutem Erfolg eingesetzt werden. Dabei steht n je nach der Ladung des im quaternisierten Produkt vorliegenden Anions A für die Zahl 1 oder 2.

Die Quaternierung der erfindungsgemäß verwendeten Homo- oder Copolymerisate erfolgt nach dem Polymerisationsschritt oder vorzugsweise vor dem Polymerisationsschritt, d.h. man setzt bereits quaternisierte Monomere A ein. Beispiele hierfür sind 3-Methyl-1-vinylimidazoliumcarbonat, 3-Methyl-1-vinylimidazoliumjodid, 2,3-Dimethyl-1-vinylimidazoliummethylsulfat, 3-Ethyl-2-methyl-1-vinylimidazoliumethylsulfat, 3-Ethyl-1-vinylimidazoliumethylsulfat, 3-Methyl-1-vinylimidazoliummethylsulfat, 2,3-Dimethyl-1-vinylimidazoliumjodid, 3-n-Dodecyl-1-vinylimidazoliumjodid, 3-n-Octadecyl-1-vinylimidazoliumjodid und 3-n-Hexadecyl-1-vinylimidazoliummjodid.

Bevorzugt eingesetzt wird 3-Ethyl- oder 3-Methyl-1-vinylimidazoliummethylsulfat oder das entsprechende Jodid.

Die Quaternierung der Monomere A und der entsprechenden Copolymerisate mit den Quaternierungsmitteln II erfolgt nach üblichen Methoden. Man arbeitet hierbei üblicherweise in wäßrigen, alkoholischen oder wäßrig-alkoholischen Lösungen, z.B. Wasser-Methanol, Wasser-Ethanol oder Wasser-Isopropanol, und bei Temperaturen kleiner als 50°C.

Zweckmäßige mit A copolymerisierbare Monomere B sind wasserlösliche ungesättigte Monomere, vor allem nicht quaternisierte 1-Vinylimidazole im Falle, daß man von bereits quaternisierten Monomeren A ausgeht, Acrylamid, Methacrylamid, N-Methylolacrylamid, N-Methylolmethacrylamid, Hydroxylalkylacrylate und Hydroxyalkylmethacrylate mit 2 bis 4 C-Atomen im Hydroxyalkylrest wie Hydroxyethyl(meth)acrylat, gegebenenfalls in Form ihrer technischen Gemische, Polyethylenglykol(meth)acrylate mit 2 bis 50 Ethylenoxideinheiten, und insbesondere 1-Vinylamide wie 1-Vinylpyrrolidon, 1-Vinylpiperidon, 1-Vinylcaprolactam, 1-Vinylformamid, 1-Vinylacetamid oder 1-Methyl-1-vinylacetamid.

Weiterhin können als Komponente B auch wasserunlösliche Monomere wie Styrol, α-Olefine, z.B. Penten oder Buten, Acryl- und Methacrylsäurealkylester mit 1 bis 18 C-Atomen im Alkylrest, Carbonsäurevinylester mit 2 bis 10 C-Atomen in Carbonsäurerest, z.B. Vinylacetat oder Vinylpropionat, mit guaternisierten 1-Vinylimidazolen A copolymerisiert werden. Dabei ist es für die Polymerisation vorteilhaft, wenn dem Gemisch eine solche Menge eines Alkohols als Lösungsmittel zugesetzt wird, daß eine homogene Lösung entsteht.

Besonders vorteilhaft kann als Komponente B 1-Vinylpyrrolidon, 2-Hydroxyethylacrylat, 2-Hydroxyethylmethacrylat, 2-Hydroxypropylacrylat, 3-Hydroxypropylacrylat oder eine Mischung hieraus verwendet werden.

In einer bevorzugten Ausführungsform ist das erfindungsgemäß verwendete Polymerisat aus der Komponente A alleine oder aus 10 bis 98 Gew.-%, vorzugsweise 20 bis 95 Gew.-% der Komponente A in quaternierter Form und 2 bis 90 Gew.-%, vorzugsweise 5 bis 80 Gew.-% der Komponente B aufgebaut.

Die Polymerisation der Komponente A und gegebenenfalls B wird, insbesondere bei Einsatz von bereits quaternisiertem Monomeren A, zweckmäßigerweise in 10 bis 50 gew.-%igen wäßrigen oder wäßrig-alkoholischen Lösungen, z.B. in Wasser-Methanol, Wasser-Ethanol oder Wasser-Isopropanol, vorzugsweise in rein wäßrigen Lösungen, in Gegenwart von radikalbildenden Initiatoren in einer Menge von 0,01 bis 20 Gew.-%, bezogen auf das Gesamtgewicht der Monomeren, und bei Temperaturen von 40 bis 150°C, insbesondere bei 50 bis 100°C durchgeführt. Man kann die Polymerisation bei Normaldruck, unter vermindertem Druck, etwa ab 100 mbar, oder bei erhöhtem Druck, etwa bis 5 bar, vornehmen; in der Regel arbeitet man bei Normaldruck.

Als radikalbildende Initiatoren sind vorzugsweise alle diejenigen Verbindungen geeignet, die bei der jeweils gewählten Polymerisationstemperatur eine Halbwertszeit von weniger als 3 Stunden aufweisen. Falls man die Polymerisation zunächst bei niedriger Temperatur startet und bei höherer Temperatur zu Ende führt, ist es zweckmäßig, mit mindestens zwei bei verschiedenen Tempraturen zerfallenden Initiatoren zu arbeiteten, nämlich zunächst einen bereits bei niedriger Temperatur zerfallenden Initiator für den Start der Polymerisation einzusetzen und dann die Hauptpolymerisation mit einem Initiator zu Ende zu führen, der bei höherer Temperatur zerfällt. Man kann wasserlösliche sowie wasserunlösliche Initiatoren oder Mischungen von wasserlöslichen und wasserunlöslichen Initiatoren einsetzen. Die in Wasser unlöslichen Initiatoren sind dann in der organischen Phase löslich.

Für die im folgenden angegebenen Temperaturbereiche kann man beispielsweise die dafür aufgeführten Initiatoren verwenden.

### Temperatur: 40 bis 60°C

Acetylcyclohexansulfonylperoxid, Diacetylperoxidicarbonat, Dicyclohexylperoxidicarbonat, Di-2-ethylhexylperoxidicarbonat, tert.-Butylperneodecanoat, tert.-Amylperneodecanoat, 2,2'-Azobis-(4-methoxy-2,4-dimethylvaleronitril), 2,2'-Azobis-(2-amidinopropan)-dihydrochlorid, 2,2'-Azobis-[2-(2-imidazolin-2-yl)-propan]dihydrochlorid;

### Temperatur: 60 bis 80°C

tert.-Butylperpivalat, tert.-Amylperpivalat, Dioctanoylperoxid, Dilaurylperoxid, 2,2'-Azobis-(2,4-dimethylvaleronitril);

### Temperatur: 80 bis 100°C

Dibenzoylperoxid, tert.-Butylper-2-ethylhexanoat, tert.-Butylpermaleinat, 2,2'-Azobis-(isobutyronitril), Dimethyl-2,2'-azobisisobutyrat, Natriumpersulfat, Kaliumpersulfat, Ammoniumpersulfat;

### Temperatur: 100 bis 120°C

Bis-(tert.-butylperoxi)-cyclohexan, tert.-Butylperoxiisopropyl-carbonat, tert.-Butylperacetat, Wasserstoffperoxid;

### Temperatur: 120 bis 140°C

2,2-Bis-(tert.-butylperoxi)-butan, Dicumylperoxid, Ditert.-amylperoxid, Di-tert.-butylperoxid;

### Temperatur: > 140°C

p-Menthanhydroperoxid, Pinanhydroperoxid, Cumolhydroperoxid und tert.-Butylhydroperoxid.

Verwendet man zusätzlich zu den genannten Initiatoren noch Salze oder Komplexe von Schwermetallen, z.B. Kupfer-, Kobalt-, Mangan-, Eisen-, Vanadium-, Nickel- oder Chromsalze, oder organische Verbindungen, z.B. Benzoin, Dimethylanilin oder Ascorbinsäure, so können die Halbwertszeiten der angegebenen radikalbildenden Initiatoren verringert werden. So kann man beispielsweise tert.-Butylhydroperoxid unter Zusatz von 5 ppm Kupfer-III-acetylacetonat so aktivieren, daß bereits bei 100°C polymerisiert werden kann.

Die reduzierende Komponente von Redoxkatalysatoren kann beispielsweise von Verbindungen wie Natriumsulfit, Natriumformaldehydsulfoxylat oder Hydrazin gebildet werden.

Bezogen auf das bei der Polymerisation eingesetzte Gesamtgewicht der Monomeren verwendet man 0,01 bis 20, vorzugsweise 0,05 bis 10 Gew.-% eines Polymerisationsinitiators oder einer Mischung mehrerer Polymerisationsinitiatoren, als Redoxkomponenten setzt man 0,01 bis 15 Gew.-% der reduzierend wirkenden Verbindungen zu. Schwermetalle werden im Bereich von 0,1 bis 100 ppm, vorzugsweise 0,5 bis 10 ppm eingesetzt. Oft ist es von Vorteil, eine Kombination aus Peroxid, Reduktionsmittel und Schwermetall als Redoxkatalysator einzusetzen.

Das Molekulargewicht der erfindungsgemäß verwendeten Homo- oder Copolymerisate kann, falls gewünscht, durch Zugabe von Reglern in die Polymerisationsmischung modifiziert, insbesondere erniedrigt werden. Als Regler kommen beispielsweise niedere Alkohole, z.B. Methanol, Ethanol, n-Propanol, Isopropanol, n-Butanol, Isobutanol, tert.-Butanol und Isopentanol, in Betracht, sie können zweckmäßigerweise bereits bei der Herstellung der quaternisierten 1-Vinylimidazole in der erforderlichen Menge zugefügt werden. Als Regler für das Molekulargewicht können aber auch andere üblicherweise zu diesem Zweck eingesetzte Verbindungen, wie Schwefelverbindungen, beispielsweise 2-Mercaptoethanol, Butylmerkaptan, Dodecylmerkaptan, Thioessigsäure oder Thiomilchsäure, Halogenverbindungen, wie Tetrachlorkohlenstoff oder 1,1,1-Tribrompropan, oder Ameisensäure und ihre Derivate eingesetzt werden.

Durch die geeignete Wahl von Reglern, Initiatoren, der Polymerisationstemperatur und der Monomerenkonzentration wird der K-Wert des erhaltenen Polymerisats, der ein Maß für das Molekulargewicht ist, eingestellt. Die K-Werte der erhaltenen Copolymerisate betragen üblicherweise 20 bis 150 und die der Homopolymerisate 15 bis 120, wobei die Messungen an einer 1 gew.-%igen, wäßrigen Lösung bei 25°C vorgenommen werden. Bei hochviskosen Lösungen empfiehlt es sich, den K-Wert an einer 0,1 gew.-%igen wäßrigen Lösung bei 25°C zu messen; die K-Werte liegen dann für Copolymerisate zwischen 30 bis 350 und für Homopolymerisate zwischen 25 bis 230.

Homo- oder Copolymerisate auf Basis von quaternisierten 1-Vinylimidazolen, welche aus den Komponenten A und B aufgebaut sind und bei deren Herstellung Quaternierungsmittel der allgemeinen Formel II verwendet werden, sind im Prinzip aus der Literatur bekannt. So werden in der Literaturstelle ACS Polymer Preprints 13 (1), S. 271-275 (1972) (4) Homopolymerisate von Vinylimidazolium-Salzen und in der Literaturstelle ACS Polymer Preprints 15 (1), S. 463-467 (1974) (5) Copolymerisate von Vinylimidazolium-Salzen mit N-Vinylpyrrolidon, Acrylamid und Styrol beschrieben. In keiner dieser Literaturstellen werden jedoch Hinweise auf mögliche Anwendungen dieser Polymerisate oder auf anwendungstechnisch relevante Eigenschaften gegeben.

Die erhaltenen Homo- oder Copolymerisate können als organische Polyelektrolyte vielfältig eingesetzt werden. In kosmetischen Zubereitungen eignen sie sich vor allem als Haarkonditionierungsmittel, d.h. als Zusatz zu den üblichen haarkosmetischen Mitteln zur Verbesserung von Kämmbarkeit, Glanz und Weichheit des Haars und zur Verringerung dessen elektrostatischer Aufladung, beispielsweise in Schaumkonditionern, Schaumfestigern, Festigerlösungen, Festigergelen oder Haarspülungen; die weiteren üblichen Bestandteile solcher haarkosmetischer Zubereitungen sind dem Fachmann bekannt und brauchen deshalb hier nicht weiter erläutert werden. Die erhaltenen Homo- oder Copolymerisate eignen sich weiterhin als Leitfähigkeitsharze in Mitteln zur Leitfähigkeitserhöhung, z.B. bei der Beschichtung von Tischler- und Spanplatten für Büro- und Computermöbel zur Herabsetzung der elektrostatischen Aufladbarkeit. Die erhaltenen Homo- oder Copolymerisate eignen sich weiterhin als wirksamer Bestandteil in Flokkungsmitteln, z.B. für die Reinigung von industriellen und kommunalen Abwässern, die Aufbereitung von Trinkwasser oder die Gewinnung von Steinsalz, Steinkohle, Kaolin und Erzen durch Flotation. Die erhaltenen Homo- oder Copolymerisate eignen sich weiterhin als wirksamer Bestandteil in Hilfsmitteln bei der Erdölgewinnung, z.B. zur Entfernung von Restölanteilen aus Wasser in Raffinerien, als Schutzkolloide für Bohrspülungen und Bohrlochzementierungen, als Emulsionsspalter zur Entwässerung von Rohölemulsionen oder zur Entsalzung von Rohölen.

Wie aus den nachfolgenden Beispielen ersichtlich, verursachen die erfindungsgemäß verwendeten Homo- oder Copolymerisate praktisch keine Korrosion bei Stählen und sonstigen üblichen Eisenlegierungen und weisen gleichzeitig eine hohe Ladungsdichte und eine hohe Hydrolysestabilität auf, was für ihre Anwendungen als Polyelektrolyt-Systeme von besonderer Bedeutung ist.

Die folgenden Beispiele erläutern die Erfindung. Der K-Wert wurde entweder an einer 1 gew.-%igen oder 0,1 gew.-%igen Lösung in Wasser bei 25°C gemäß Fikentscher gemessen (K. Fikentscher, Cellulose-Chemie, Band 13, S. 58 bis 64 und 71 bis 74, 1932).

### Beispiel 1

Eine Mischung aus 280 g 3-Methyl-1-vinylimidazoliummethylsulfat, 15 g N-Vinylpyrrolidon und 400 g Wasser wurde mit 10 gew.-%iger Natronlauge auf einen pH-Wert von 7,5 eingestellt (Zulauf 1). Aus 2,5 g 2,2'-Azobis-(2-methylpropionamidin)dihydrochlorid und 100 g Wasser wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 12 ml Zulauf 2 vorgelegt und unter Rühren auf 65°C erhitzt. Bei dieser Temperatur wurden der restliche Zulauf 1 innerhalb von 5 Stunden und der restliche Zulauf 2 innerhalb von 7 Stunden zudosiert. Anschließend wurde noch eine Stunde lang bei dieser Temperatur gerührt. Man erhielt eine klare, viskose Polymerlösung. Der K-Wert des Polymeren betrug 110,5 (1 gew.-%ig).

### Beispiel 2

Eine Mischung aus 120 g 3-Methyl-1-vinylimidazoliummethylsulfat, 120 g N-Vinylpyrrolidon und 380 g Wasser wurde mit 10 gew.-%iger Natronlauge auf einen pH-Wert von 7,5 eingestellt (Zulauf 1). Aus 2,5 g 2,2'-Azobis-(2-methylpropionamidin)dihydrochlorid und 100 g Wasser wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 12 ml Zulauf 2 vorgelegt und unter Rühren auf 60°C erhitzt. Bei dieser Temperatur wurden der restliche Zulauf 1 innerhalb von 7 Stunden und der restliche Zulauf 2 innerhalb von 9 Stunden zudosiert. Anschließend wurde noch eine Stunde lang bei dieser Temperatur gerührt. Man erhielt eine klare, viskose Polymerlösung. Der K-Wert des Polymeren betrug 312,5 (0,1 gew.-%ig) bzw. 133,2 (1 gew.-%).

### Beispiel 3

Eine Mischung aus 120 g 3-Methyl-1-vinylimidazoliumjodid, 120 g N-Vinylpyrrolidon und 380 g Wasser wurde mit 10 gew.-%iger Natronlauge auf einen pH-Wert von 7,5 eingestellt (Zulauf 1). Aus 3,5 g 2,2'-Azobis-(2-methylpropionamidin)dihydrochlorid und 100 g Wasser wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 12 ml Zulauf 2 vorgelegt und unter Rühren auf 60°C erhitzt. Bei dieser Temperatur wurden der restliche Zulauf 1 innerhalb von 5 Stunden und der restliche Zulauf 2 innerhalb von 6 Stunden zudosiert. Anschließend wurde noch zwei Stunden lang bei dieser Temperatur gerührt. Man erhielt eine klare, viskose Polymerlösung. Der K-Wert des Polymeren betrug 288,7 (0,1 gew.-%ig).

### Beispiel 4

Eine Mischung aus 48 g 3-Methyl-1-vinylimidazoliummethylsulfat, 192 g N-Vinylpyrrolidon und 350 g Wasser wurde mit 10 gew.-%iger Natronlauge auf einen pH-Wert von 7,8 eingestellt (Zulauf 1) . Aus 3,0 g 2,2'-Azobis-(2-methylpropionamidin)dihydrochlorid und 100 g Wasser wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 12 ml Zulauf 2 vorgelegt und unter Rühren auf 60°C erhitzt. Bei dieser Temperatur wurden der restliche Zulauf 1 innerhalb von 4 Stunden und der restliche Zulauf 2 innerhalb von 6 Stunden zudosiert. Anschließend wurde noch eine Stunde lang bei dieser Temperatur gerührt. Man erhielt eine klare, viskose Polymerlösung. Der K-Wert des Polymeren betrug 300,4 (0,1 gew.-%ig).

### Beispiel 5

Eine Mischung aus 24 g 3-Methyl-1-vinylimidazoliummethylsulfat, 216 g N-Vinylpyrrolidon und 480 g Wasser wurde mit 10 gew.-%iger Natronlauge auf einen pH-Wert von 7,3 eingestellt (Zulauf 1). Aus 3,0 g tert.-Butylpernedecanoat und 20 g Ethanol wurde Zulauf 2 hergestellt. In einem 2 l-Rührbehälter, der mit Rührer, Heizung, Rückflußkühler und Dosiervorrichtung ausgestattet war, wurden 300 g Wasser, 120 ml Zulauf 1 und 2,5 ml Zulauf 2 vorgelegt und unter Rühren auf 55°C erhitzt. Bei dieser Temperatur wurden der restliche Zulauf 1 innerhalb von 5 Stunden und der restliche Zulauf 2 innerhalb von 7 Stunden zudosiert. Anschließend wurde noch eine Stunde lang bei dieser Temperatur gerührt. Man erhielt eine klare, viskose Polymerlösung. Der K-Wert des Polymeren betrug 297,2 (0,1 gew.-%ig).

### Beispiel 6

Eine Mischung aus 240 g 3-Methyl-1-vinylimidazoliummethylsulfat und 380 g Wasser wurde mit 10 gew.-%iger Natronlauge auf einen pH-Wert von 7,9 eingestellt (Zulauf 1). Aus 4,2 g tert.-Amylperneodecanoat und 20 g Ethanol wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 2,5 ml Zulauf 2 vorgelegt und unter Rühren auf 53°C erhitzt. Bei dieser Temperatur wurden der restliche Zulauf 1 innerhalb von 6 Stunden und der restliche Zulauf 2 innerhalb von 8 Stunden zudosiert. Anschließend wurde noch eine Stunde lang bei 65°C gerührt. Nach dem Abkühlen erhielt man erhielt eine klare, gelbliche, viskose Polymerlösung. Der K-Wert des Polymeren betrug 103,2 (1 gew.-%ig).

### Beispiel 7

Eine Mischung aus 120 g 3-Methyl-1-vinylimidazoliummethylsulfat, 120 g N-Vinylpyrrolidon, 1,2 g Mercaptoethanol und 350 g Wasser wurde mit konzentrierter Ammoniaklösung auf einen pH-Wert von 7,5 eingestellt (Zulauf 1). Aus 3,0 g 2,2'-Azobis-(2-methylpropionamidin)dihydrochlorid und 100 g Wasser wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 12 ml Zulauf 2 vorgelegt und unter Rühren auf 55°C erhitzt. Bei dieser Temperatur wurden der restliche Zulauf 1 innerhalb von 7 Stunden und der restliche Zulauf 2 innerhalb von 9 Stunden zudosiert. Anschließend wurde noch eine Stunde lang bei dieser Temperatur gerührt. Man erhielt eine klare, viskose Polymerlösung. Der K-Wert des Polymeren betrug 82,3 (1 gew.-%ig).

### Beispiel 8

Eine Mischung aus 72 g 3-Methyl-1-vinylimidazoliummethylsulfat gelöst in 88 g Wasser, 168 g N-Vinylpyrrolidon und 300 g Wasser wurde mit 10 gew.-%iger Natronlauge auf einen pH-Wert von 7,6 eingestellt (Zulauf 1) . Aus 3,1 g tert.-Butylperacetat, gelöst in 20 g Ethanol, wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 2,5 ml Zulauf 2 vorgelegt und unter Rühren bis zum Sieden erhitzt. Bei dieser Temperatur wurden der restliche Zulauf 1 innerhalb von 5 Stunden und Zulauf 2 innerhalb von 7 Stunden zudosiert. Anschließend wurde noch eine Stunde unter Rückfluß gerührt. Nach dem Abkühlen erhielt man eine klare, viskose Polymerlösung. Der K-Wert des Polymeren betrug 203,7 (0,1 gew.-%ig).

### Beispiel 9

Eine Mischung aus 96 g 3-Ethyl-1-vinylimidazoliumethylsulfat gelöst in 120 g Wasser, 144 g N-Vinylpyrrolidon und 300 g Wasser wurde mit 10 gew.-%iger Natronlauge auf einen pH-Wert von 7,5 eingestellt (Zulauf 1). Aus 2,8 g tert.-Butylperpivalat, gelöst in 20 g Ethanol, wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 2,5 ml Zulauf 2 vorgelegt und unter Rühren bis auf 65°C erhitzt. Bei dieser Temperatur wurden der restliche Zulauf 1 innerhalb von 5 Stunden und Zulauf 2 innerhalb von 7 Stunden zudosiert. Anschließend wurde noch eine Stunde unter Rückfluß gerührt. Nach dem Abkühlen erhielt man eine klare, viskose Polymerlösung. Der K-Wert des Polymeren betrug 289,2 (0,1 gew.-%ig).

### Beispiel 10

Eine Mischung aus 120 g 3-Ethyl-1-vinylimidazoliumethylsulfat gelöst in 150 g Wasser, 120 g 2-Hydroxyethylacrylat und 250 g Wasser wurde hergestellt (Zulauf 1). Aus 2,9 g tert.-Amylper-2-ethylhexanoat und 30 g Ethanol wurde Zulauf 2 hergestellt. In einem 2 l-Rührbehälter, der mit Rührer, Heizung, Rückflußkühler und Dosiervorrichtung ausgestattet war, wurden 300 g Wasser, 100 ml Zulauf 1 und 4 ml Zulauf 2 vorgelegt und unter Rühren bis auf 85°C erhitzt. Bei dieser Temperatur wurden der restliche Zulauf 1 innerhalb von 5 Stunden und Zulauf 2 innerhalb von 7 Stunden zudosiert. Anschließend wurde noch eine Stunde unter Rückfluß gerührt. Nach dem Abkühlen erhielt man eine klare, viskose Polymerlösung. Der K-Wert des Polymeren betrug 88,2 (0,1 gew.-%ig).

### Beispiel 11

Eine Mischung aus 120 g 2,3-Dimethyl-1-vinylimidazoliummethylsulfat, 120 g N-Vinylpyrrolidon und 400 g Wasser wurde mit 10 gew.-%iger Natronlauge auf einen pH-Wert von 7,5 eingestellt (Zulauf 1). Aus 2,7 g Di-tert.-butylperoxid und 30 g Ethanol wurde Zulauf 2 hergestellt. In einem 1,5 l-Druckbehälter, der mit Rührer, Heizung, Rückflußkühler und Dosiervorrichtung ausgestattet war, wurden 300 g Wasser, 100 ml Zulauf 1 und 4 ml Zulauf 2 vorgelegt und unter Rühren bis auf 135°C erhitzt. Dabei baute sich ein Druck von 3,1 atm auf. Bei 135°C wurde der restliche Zulauf 1 innerhalb von 5 Stunden und Zulauf 2 innerhalb von 7 Stunden zudosiert. Anschließend wurde noch eine Stunde bei dieser Temperatur gerührt. Man erhielt eine klare, gelbliche, viskose Polymerlösung. Der K-Wert des Polymeren betrug 85,3 (1 gew.-%ig).

### Beispiel 12

Eine Mischung aus 218 g 3-Methyl-1-vinylimidazoliummethylsulfat, gelöst in 280 g Wasser, 12 g N-Vinylpyrrolidon und 150 g Wasser wurde mit 10 gew.-%iger Natronlauge auf einen pH-Wert von 7,4 gestellt (Zulauf 1). Aus 3,0 g 2, 2'-Azobis(2-methylpropionamidin)dihydrochlorid und 100 g Wasser wurde Zulauf 2 hergestellt. In einem 1,5 l-Druckbehälter, der mit Rührer, Heizung, Rückflußkühler und Dosiervorrichtung ausgestattet war, wurden 250 g Wasser, 100 ml Zulauf 1 und 4 ml Zulauf 2 vorgelegt und unter Rühren bis auf 55°C erhitzt. Der Druck wurde so eingestellt, daß die Reaktionsmischung siedet (ca. 200 mbar). Bei dieser Temperatur wurde der restliche Zulauf 1 innerhalb von 5 Stunden und Zulauf 2 innerhalb von 7 Stunden zudosiert. Anschließend wurde noch eine Stunde bei dieser Temperatur gerührt. Man erhielt eine klare viskose Polymerlösung. Der K-Wert des Polymeren betrug 107,3 (1 gew.-%ig).

### Beispiel 13

Es wurde das Korrosionsverhalten der Stähle Nr. 1.4541 (V₂A-Stahl) und 1.4571 (V₄A-Stahl) bei 70°C in Wasser untersucht. Die Versuchszeit betrug 3 Wochen; nach jeweils einer Woche wurde das Medium erneuert. Ferner wurde eine elektrochemische Untersuchung auf Lochfraßgefahr durchgeführt. Tabelle 1 zeigt die Ergebnisse.

**Tabelle 1**

| | | lineare Korrosionsgeschwindigkeit pro Jahr in mm pro Jahr | |
|---|---|---|---|
| | | V₂A-Stahl | V₄A-Stahl |
| Zum Vergleich: | | | |
| Beispiel 1 | aus | 0,023 (Lochfraßgefahr) | 0,025 (Lochfraßgefahr) |
| (1) | | | |
| Beispiel 6 | aus | 0,02 | 0,02 (Lochfraßgefahr) |
| (1) | | | |
| Beispiel 13 | aus | 0,015 (Lochfraßgefahr) | 0,018 |
| (1) | | | |
| erfindungsgemäß: | | | |
| Beispiel 1 | | < 0,001 | < 0,001 |
| Beispiel 6 | | < 0,001 | < 0,001 |

### Beispiel 14

### Ladungsdichte

Die Ladungsdichte wurde ermittelt nach dem Verfahren von D. Horn, beschrieben in Progr. Colloid and Polym. Sci. 65, S. 251-264 (1978). Diese Methode ist ein automatisiertes kolorimetrisches Titrierverfahren zur Bestimmung von organischen Polyelektrolyten in wäßriger Lösung und beruht auf der Komplexbildung zwischen kationischen und anionischen Polymeren. Tabelle 2 zeigt die Ergebnisse.

**Tabelle 2**

| Beispiele | Ladungsdichte (bez. auf Feststoff, bei 20°C und pH 7) in meq/g |
|---|---|
| erfindungsgemäß: | |
| Bsp. 1 | 4,3 |
| Bsp. 2 | 2,3 |
| Bsp. 6 | 4,6 |
| Zum Vergleich: | |
| Bsp. 6 aus (1) | 6,5 |
| Bsp. 5 aus (1) | 2,1 |
| Bsp. 13 aus (1) | 3,4 |
| Bsp. 6 aus (2) | 0,7 |
| Bsp. 7 aus (2) | 1,0 |
| Bsp. 1 aus (3) | 0,3 |
| Bsp. 2 aus (3) | 0,9 |
| Bsp. 7 aus (3) | 0,6 |

### Beispiel 15

### Hydrolyseversuche

Tabelle 3 zeigt die Ergebnisse von Hydrolyseversuchen, bei denen der prozentuale Anteil an hydrolysiertem Material in Abhängigkeit von der Zeit bestimmt wurde.

**Tabelle 3**

| | Temperatur °C | pH-Wert | % Hydrolyse Monate | | | |
|---|---|---|---|---|---|---|
| | | | 1 | 2 | 3 | 4 |
| erfindungsgemäß Beispiel 2 | 45 | 10 | 0 | 0 | 0 | 0 |
| | 25 | 10 | 0 | 0 | 0 | 0 |
| | 45 | 7 | 0 | 0 | 0 | 0 |
| | 25 | 7 | 0 | 0 | 0 | 0 |
| zum Vergleich: Beispiel 6 aus (3) | 45 | 10 | 5 | 3 | - | 10 |
| | 25 | 10 | 5 | 3 | - | 4 |
| | 45 | 7 | 5 | 10 | - | 16 |
| | 25 | 7 | 5 | 10 | - | 8 |
| zum Vergleich: Beispiel 24 aus (3) | 45 | 10 | 30 | - | 51,0 | 90 |
| | 25 | 10 | 14 | - | 14,4 | 30,6 |
| | 45 | 7 | 8,5 | - | 15,6 | 22,0 |
| | 25 | 7 | 0 | - | 0 | 4,8 |
| erfindungsgemäß Beispiel 6 | 45 | 10 | 0 | 0 | 0 | 0 |
| | 25 | 10 | 0 | 0 | 0 | 0 |
| | 45 | 7 | 0 | 0 | 0 | 0 |
| | 25 | 7 | 0 | 0 | 0 | 0 |
| zum Vergleich: Beispiel 13 aus (1) | 45 | 10 | 0 | 0 | 0 | 0 |
| | 25 | 10 | 0 | 0 | 0 | 0 |
| | 45 | 7 | 0 | 0 | 0 | 0 |
| | 25 | 7 | 0 | 0 | 0 | 0 |

## Patentansprüche

1. Verwendung von Homo- oder Copolymerisaten auf Basis von quaternisierten 1-Vinylimidazolen, welche aus
A) 10 bis 100 Gew.-% eines 1-Vinylimidazols der allgemeinen Formel I 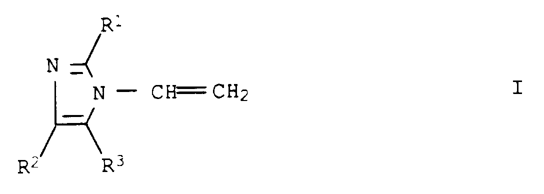 in der R¹ bis R³ für Wasserstoff oder C₁- bis C₄-Alkyl stehen, und
B) 0 bis 90 Gew.-% weiterer copolymerisierbarer Monomerer, jeweils bezogen auf die Summe aus A in quaternisierter Form und B,
aufgebaut sind, als organische Polyelektrolyte in kosmetischen Zubereitungen, in Mitteln zur Leitfähigkeitserhöhung, in Flockungsmitteln und in Hilfsmitteln bei der Erdölgewinnung, dadurch gekennzeichnet, daß man solche Homo- oder Copolymerisate einsetzt, bei deren Herstellung als Quaternierungsmittel Verbindungen der allgemeinen Formel II
(R⁴) ₙA II
in der
R⁴ für C₁- bis C₂₀-Alkyl oder Benzyl steht,
A Jodid, Carbonat, Methylsulfat, Ethylsulfat oder Sulfat bezeichnet und
n die Zahl 1 oder 2 bedeutet,
verwendet wurden.

2. Verwendung von Homo- oder Copolymerisaten auf Basis von quaternisierten 1-Vinylimidazolen nach Anspruch 1, dadurch gekennzeichnet, daß man solche Homo- oder Copolymerisate einsetzt, bei deren Herstellung als Quaternierungsmittel Methyljodid, Dimethylsulfat oder Diethylsulfat verwendet wurden.

3. Verwendung von Homo- oder Copolymerisaten auf Basis von quaternisierten 1-Vinylimidazolen nach Anspruch 1 oder 2, wobei das Polymerisat aus unsubstituiertem 1-Vinylimidazol als Komponente A aufgebaut ist.

4. Verwendung von Copolymerisaten auf Basis von quaternisierten 1-Vinylimidazolen nach den Ansprüchen 1 bis 3, wobei das Copolymerisat aus 1-Vinylpyrrolidon, 2-Hydroxyethylacrylat, 2-Hydroxyethylmethacrylat, 2-Hydroxypropylacrylat, 3-Hydroxypropylacrylat oder einer Mischung hieraus als Komponente B aufgebaut ist.

5. Verwendung von Homo- oder Copolymerisaten auf Basis von quaternisierten 1-Vinylimidazolen nach den Ansprüchen 1 bis 4, wobei das Polymerisat aus der Komponente A alleine oder aus 10 bis 98 Gew.-% der Komponente A in quaternisierter Form und 2 bis 90 Gew.-% der Komponente B aufgebaut ist.

## Claims

1. Use of a homo- or copolymer based on a quaternized 1-vinylimidazole, which is composed of
A) from 10 to 100% by weight of a 1-vinylimidazole of the formula I 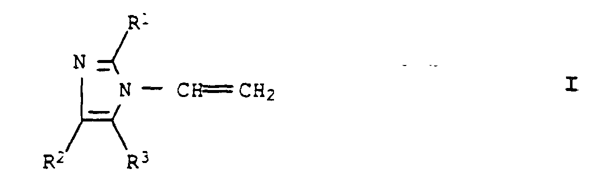 where R¹ to R³ are each hydrogen or C₁-C₄-alkyl, and
B) from 0 to 90% by weight, based in each case on the sum of A in quaternized form and B, of further copolymerizable monomers,
as an organic polyelectrolyte in cosmetic formulations, in agents for increasing conductivity, in flocculants and in assistants for oil production, wherein the homo- or copolymer used is one which has been prepared using, as quaternizing agent, a compound of the formula II
(R⁴)ₙA II
where R⁴ is C₁-C₂₀-alkyl or benzyl, A is iodide, carbonate, methyl sulfate, ethyl sulfate or sulfate and n is 1 or 2.

2. Use of a homo- or copolymer based on a quaternized 1-vinylimidazole as claimed in claim 1, wherein the homo- or copolymer used is one which has been prepared using, as quaternizing agent, methyl iodide, dimethyl sulfate or diethyl sulfate.

3. Use of a homo- or copolymer based on a quaternized 1-vinylimidazole as claimed in claim 1 or 2, wherein the polymer is composed of unsubstituted 1-wherein the polymer is composed of unsubstituted 1-vinylimidazole as component A.

4. Use of a copolymer based on a quaternized 1-vinylimidazole as claimed in any of claims 1 to 3, wherein the copolymer is composed of 1-vinylpyrrolidone, 2-hydroxyethyl acrylate, 2-hydroxyethyl methacrylate, 2-hydroxypropyl acrylate, 3-hydroxypropyl acrylate or a mixture thereof as component B.

5. Use of a homo- or copolymer based on a quaternized 1-vinylimidazole as claimed in any of claims 1 to 4, wherein the polymer is composed of component A alone or of from 10 to 98% by weight of component A in quaternized form and from 2 to 90% by weight of component B.

## Revendications

1. Utilisation, comme polyélectrolytes organiques dans des préparations cosmétiques, dans des moyens pour élever la conductibilité dans des moyens de flocage et dans des auxiliaires pour exploitation pétrolière, d'homo- ou copolymérisats à base de 1-vinylamidazoles quaternisés qui sont formés de
A) 10 à 100 % en poids d'un 1-vinylimidazole de formule générale I 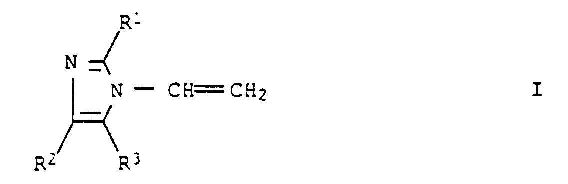 dans laquelle R¹ à R³ sont mis pour hydrogène ou alkyle en C1- à C4
B) 0 à 90 % en poids d'autres monomères copolymérisables, pourcentages rapportés chacun à la somme de A sous forme quaternisé et de B
caractérisé par le fait que l'on utilise des homo- ou copolymérisats pour la préparation desquels sont utilisés, comme agent de quaternisation, des composés de formule générale II
(R⁴)ₙA II
dans laquelle
R⁴ est mis pour alkyle en C1 à C20 ou benzyle
A représente iodure, carbonate, sulfate de méthyle, sulfate d'éthyle ou sulfate et
n représente le nombre 1 ou 2.

2. Utilisation d'homo- ou copolymérisats à base de 1-vinylimidazoles quaternisés selon la revendication 1, caractérisée par le fait qu'on incorpore des homo- ou copolymérisats tels que, lors de leur préparation, ont été utilisés, comme agent de quaternisation, du iodure de méthyle, du sulfate de diméthyle ou du sulfate de diéthyle.

3. Utilisation d'homo- ou copolymérisats à base de 1-vinylimidazoles quaternisés selon la revendication 1 ou 2, le polymérisat étant composé de 1-vinylimidazole non substitué, comme composant A.

4. Utilisation d'homo- ou copolymérisats à base de 1-vinylimidazoles quaternisés selon les revendications 1 à 3, le copolymérisat étant formé, comme composant B, de 1-vinylpyrrolidone, 2-hydroxyéthylacrylate, 2-hydroxyéthylméthacrylate, 2-hydroxypropylacrylate, 3-hydroxypropylacrylate ou d'un mélange de ceux-ci.

5. Utilisation d'homo- ou copolymérisats à base de 1-vinylimidazoles quaternisés selon les revendications 1 à 4, le polymérisat étant formé du composant A seul ou de 10 à 98 % en poids du composant sous forme quaternisée et 2 à 90 % en poids du composant B.
